# EUROPÄISCHE PATENTSCHRIFT

(11) **EP 0 910 275 B1**
(45) Veröffentlichungstag und Bekanntmachung des Hinweises auf die Patenterteilung: **01.12.1999**
(21) Anmeldenummer: 96916126.4
(22) Anmeldetag: 18.05.1996
(51) Int. Cl.: A47L 13/17

(54) **OFFENPORIGER, FLEXIBLER REINIGUNGSKÖRPER**
OPEN-PORE FLEXIBLE CLEANING MEMBER
CORPS NETTOYEUR SOUPLE ET A PORES OUVERTS

(43) Veröffentlichungstag der Anmeldung: 28.04.1999
(73) Patentinhaber: Firma Carl Freudenberg, 69469 Weinheim (DE)
(72) Erfinder: TINTELNOT, Carl-Uwe, D-69469 Weinheim (DE)
(86) Internationale Anmeldenummer: EP9602149
(87) Internationale Veröffentlichungsnummer: WO9700001

(56) Entgegenhaltungen:
- EP-A- 0 047 797
- EP-A- 0 458 655
- GB-A- 946 634
- US-A- 3 226 753
- US-A- 3 965 519

## Beschreibung

### Technisches Gebiet

Die Erfindung betrifft einen offenporigen, flexiblen Reinigungskörper mit zumindest einer Scheuerfläche, die zumindest in einem Teilbereich mit über eine Deckfläche (4) mit einer Höhe (C,D) erhaben vorstehenden kontinuierlich durchgehend ausgebildeten Leisten versehen ist.

### Stand der Technik

Ein solcher Reinigungskörper ist aus dem deutschen Gebrauchsmuster 7 612 130 bekannt. Die Leisten sind dabei auf Ihrer ganzen Länge von übereinstimmendem Querschnitt, einander dicht benachbart zugeordnet und durch senkrecht eingeschnittene Kanäle voneinander getrennt. Sie bestehen aus demselben Werkstoff wie der Reinigungskörper und haben eine dementsprechend große Flexibilität. Beim seitlichen Auftreffen auf fester anhaftenden Schmutz ist häufig ein seitliches Ausknicken zu beobachten, was wenig dazu beiträgt, den Schmutz zu lösen und ihn nachfolgend zu entfernen. Außerdem neigen in die Zwischenräume der Leisten eindringende, bereits gelöste Schmutzbestandteile dazu, sich dort festzusetzen. Sie sind schwierig zu beseitigen.

Aus der DE-A-27 30 266 sind Reinigungskörper bekannt, bei denen auf einer ebenen Fläche eine Vielzahl von isolierten, durch rillenförmige Einschnitte in die Arbeitsfläche erzeugten Vorsprüngen dicht benachbart angeordnet ist. Die Vorsprünge erheben sich über eine Bodenfläche, die durch die rillenförmigen Einschnitte entstanden ist, Sodaß keine Leisten im eigentlichen Sinn vorhanden sind. Derartige Reinigungskörper gestatten es nicht, eine zu reinigende Fläche streifenfrei abzuwischen. Außerdem sinkt die Ausknickfestigkeit der Vorspünge mit zunehmender Höhe ab. Um festhaftenden Schmutz entfernen zu können ist es daher nötig, den Vorsprüngen eine sehr geringe Höhe zugeben. Dabei muß allerdings eine Beeinträchtigung der Schmutzaufnahmekapazität der Zwischenräume inkauf genommen werden.

Der Erfindung liegt die Aufgabe zugrunde, einen Reinigungskörper der eingangs genannten Art derart weiterzuentwickeln, daß die mechanische Abtragung festhaftender Schmutzbestandteile besser als bisher gelingt.

### Darstellung der Erfindung

Diese Aufgabe wird erfindungsgemäß bei einem Reinigungskörper der eingangs genannten Art mit den kennzeichnenden Merkmalen von Anspruch 1 gelöst. Auf vorteilhafte Ausgestaltungen nehmen die Unteransprüche Bezug.

Bei dem erfindungsgemäßen Reinigungskörper ist es vorgesehen, daß die Leisten kontinuierlich durchgehend ausgebildet sind und in Richtung ihres Verlaufs Bereiche einer voneinander abweichenden Höhe haben. Dabei geht die Erfindung aus von der Erkenntnis, daß Verschmutzungen eine zu reinigenden Fläche gewöhnlich mit einer weitgehend übereinstimmenden Dicke überziehen. Ihre Beseitigung wird unter Verwendung des erfindungsgemäßen Reinigungskörpers nicht in einem einzigen Schritt angestrebt, sondern dadurch, daß die Leisten jeweils nur mit ihren am weitesten vorstehenden Stellen mit der Verschmutzung in Eingriff gebracht werden, was es gestattet, an diesen Stellen große spezifische Anpreßkräfte über die Leisten auf die Verschmutzung auszuüben. Das Aufbrechen und Abtragen der Verschmutzung an isolierten Stellen bzw. beim Vorwärtsbewegen des Reinigungskörpers im Zuge isolierter Streifen, wird dadurch erleichtert. Hierbei ist es von erheblichem Vorteil, daß die erhabenen Stellen der Leisten durch weniger weit vorstehenden Zonen miteinander verbunden und dadurch in der Lage sind, auf ihrer ganzen Länge einen ergänzenden Scheibenwischereffekt zu bewirken. Beim Hin- und Herbewegen des Scheuerkörpers über eine zu reinigende Fläche in dicht benachbarten Streifen und im wesentlichen quer oder schräg zur Richtung der Leisten wird dadurch in kurzer Zeit eine vollständige Entfernung aller daran anhaftenden Schmutzbestandteile erreicht. Zusätzlich bewirken die sich im Zuge der Leisten an deren Vorsprünge anschließenden Bereiche von geringerer Höhe eine statische Abstützung der Vorsprünge gegen seitliches Ausknicken. Der Reinigungskörper eignet sich dadurch ganz besonders gut für eine Verwendung im häuslichen und gewerblichen Bereich.

Die Leisten erstrecken sich normalerweise parallel zueinander. Sie können dabei geradlinig, schlangenlinienförmig oder zickförmig verlaufend ausgebildet sein. Eine Ausildung, bei der die einander benachbarten Leisten zumindest hinsichtlich ihrer Erhebungen auf Lücke zugeordnet sind, ist ebenfalls möglich und kann die Reinigungswirkung verbessern. Die Leisten können in ihrem Verlauf unregelmäßig aufeinander folgend die Richtung ändern oder in sich selbst auslaufend und beispielsweise ring- oder ellipsenförmig förmig gestaltet sein.

Hinsichtlich seiner äußeren Gestalt kann der Reinigungskörper vielfältig modifiziert sein. Es ist beispielsweise möglich, ihm die Gestalt eines Reinigungstuches oder eines Scheuerschwamms von quaderförmiger Gestalt zu geben.

Auch hinsichtlich der zur Herstellung des Reinigungskörpers verwendeten Werkstoffe bestehen vielfältige Möglichkeiten. Neben offenzelligen Schaumstoffen können Fasern enthaltende Materialien zur Anwendung gelangen, beispielsweise Vliesstoffe oder Verbundwerkstoffe, die gegebenenfalls sowohl Schaumstoffe als auch Faserstoffe enthalten. Die Schaumund Faserstoffe können dabei eine ineinander übergehende Struktur haben bzw. ineinander eingebettet sein.

Sofern Faserstoffe zur Anwendung gelangen ist lediglich darauf zu achten, daß die Fasern keinen zu geringen Titer haben. Zweckmäßigerweise sollte der Titer im Bereich grober Stapelfasern liegen bei etwa bis dtex. Auch eine ergänzende oder alternative Verwendung von metallischen Fasern kann mit in die Überlegungen mit einbezogen werden.

Die Verwendung und Herstellung des Reinigungskörpers ist besonders einfach, wenn die Bereiche, in denen die Leisten eine unterschiedliche Höhe haben, regelmäßig wiederkehrend aufeinander folgen.

Nach einer vorteilhaften Ausgestaltung ist es vorgesehen, daß die Bereiche unterschiedlicher Höhe gleichmäßig ineinander übergehend ausgebildet sind. Hierdurch läßt sich eine Streifenbildung beim Abwischen glatter Flächen besonders einfach vermeiden. Bevorzugt werden Leisten, bei denen die Bereiche einer unterschiedlichen Höhe sinusförmig aufeinander folgen.

Um eine von der Richtung der Streifen unabhängige Verwendung des Reinigungskörpers bei Abwischprozessen zu ermöglichen kann es vorgesehen sein, daß die Leisten einander überkreuzen. Bei Verwendung eines regelmäßigen Musters besteht dabei die Möglichkeit, die Leisten abfallfrei zu erzeugen, in dem eine dickere Schicht des den Reinigungskörper bildenden Werkstoffes unter Verwendung von unnachgiebigen, reliefartig strukturierten Formkörpern elastisch deformiert und in zwei identische Reinigungskörper gespalten wird.

Die mechanische Widerstandsfähigkeit der Leisten gegen eine seitliche Ausknickbewegung läßt sich vergrößern, wenn sie, in Querrichtung betrachtet, durch geneigte Flächen begrenzt sind. Diese Flächen können, in Querrichtung betrachtet, eine Wölbung und gegebenenfalls ein sinusförmig ineinander übergehendes Profil haben.

Die Leisten sind einstückig mit dem Scheuerkörper aus demselben Material gefertigt. Die mechanische Widerstandsfähigkeit ist dementsprechend bei ungünstiger Profilierung unter Umständen sehr gering. Um diesbezüglich eine Verbesserung zu erzielen hat es sich als vorteilhaft bewährt, wenn das Verhältnis aus dem Mittelpunktsabstand einander benachbarter Leisten und deren maximaler Höhe etwa 4 bis 12 beträgt, vorzugsweise 6 bis 9. Auch unter Verwendung von relativ leicht verformbaren Materialien läßt sich bei Einhaltung dieses Verhältnisses ein ausgezeichneter Rakeleffekt erzielen.

Die Scheuerfläche kann mit einer die Abriebbeständigkeit verbessernden, flexiblen Beschichtung versehen sein, beispielsweise mit einer Beschichtung aus elastomerem Polyurethan. Das Eindringen in festhaftende Schmutzschichten wird hierdurch weiter verbessert. Zweckmäßigerweise wird eine solche Beschichtung in flüssigem Zustand auf die Scheuerfläche aufgebracht und nachfolgend verfestigt. In sie können gegebenenfalls Partikel eines Scheuermittels eingebettet sein, beispielsweise Partikel aus einem Gummigranulat und/oder einem abrasiven Schleifkorn.

Für die zügige Durchführung von Reinigungsvorgängen ist es äußerst vorteilhaft, wenn der Reinigungskörper während des Reinigungsvorgangs als Wasserspeicher verwendet werden kann und wenn es gelingt, das in dem Reinigungskörper enthaltene Wasser beispielsweise durch einfaches Zusammendrücken und Wiederloslassen in Richtung der Vorderseite der Scheuerfläche zu verlagern bzw. von dieser wegzusaugen. Um dieser Forderung gerecht zu werden hat es sich bewährt, wenn die Beschichtung flüssigkeitsdurchlässig und beispielsweise von Poren durchdrungen ist.

### Kurzbeschreibung der Zeichnung

Der Gegenstand der Erfindung wird nachfolgend anhand der Zeichnung weiter verdeutlicht. Es zeigen:
Fig. 1 einen typischen Reinigungskörper in einer Ansicht auf die Scheuerfläche

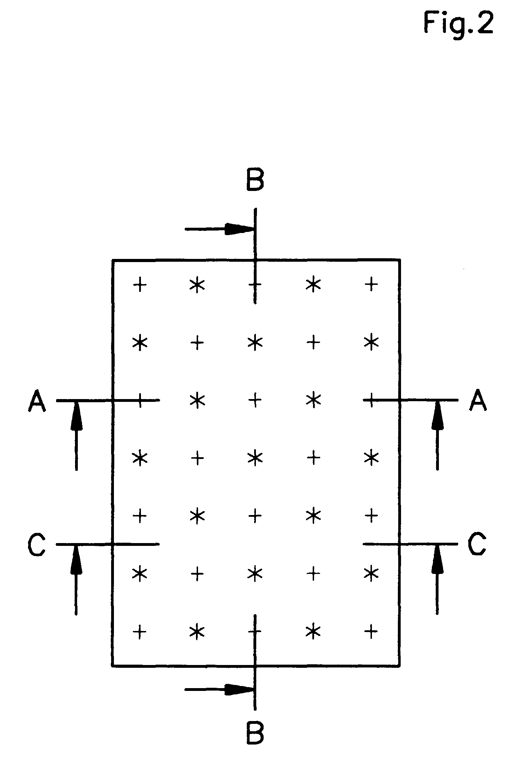

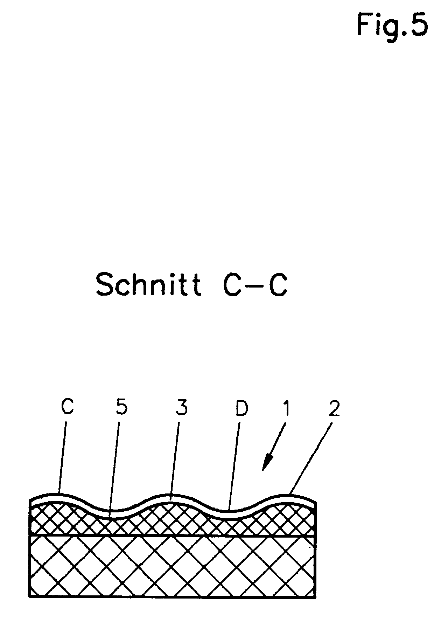

Fig. 2 eine Scheuerfläche ähnlich Fig. 1, wobei die höchsten Stellen der Leisten durch jeweils einen Stern und die niedrigsten Stellen jeweils durch eine Kreuz markiert sind und in der die Lage der einzelnen Schnittebenen nach den Fig 3 bis 5 angedeutet ist
Fig. 3 bis 5: den Reinigungskörper nach Fig. 1 in verschiedenen Schnittdarstellungen
Fig. 6 und 7 einen Reinigungskörper in quergeschnittener Darstellung und in einer Ansicht der Scheuerfläche, bei dem einander senkrecht überkreuzende Leisten sämtlich der Längsrichtung des Scheuerkörpers schräg zugeordnet sind.

### Ausführung der Erfindung

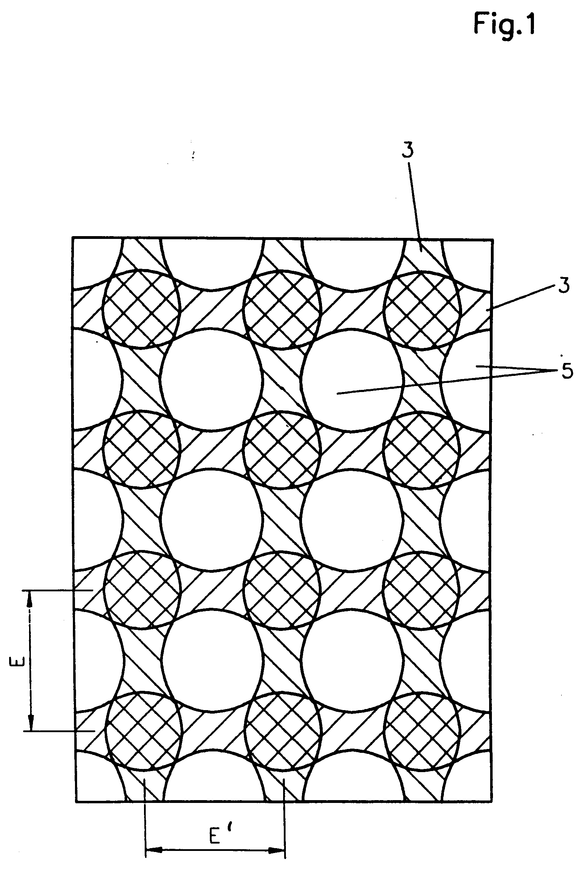

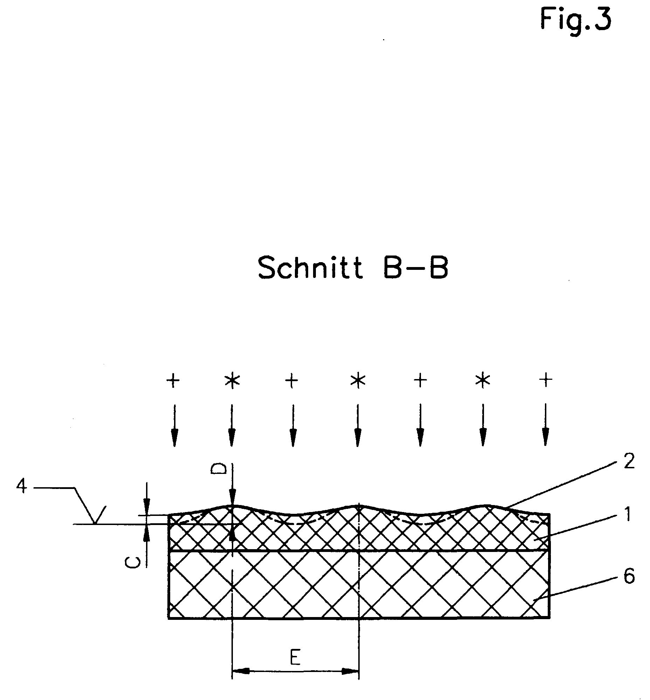

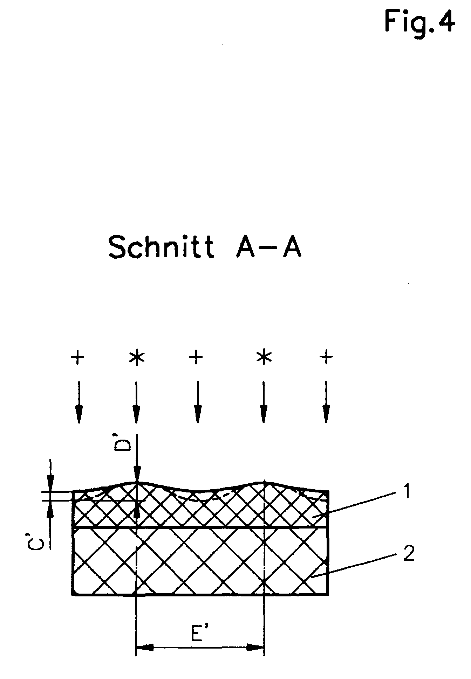

In der Zeichnung ist von einem beispielhaften Reinigungskörper die Scheuerfläche in der Draufsicht wiedergegeben. Der Reinigungsköper besteht im Bereich der Scheuerfläche 2 (Fig. 3 und 4) aus einem Schaumstoffkörper 1 aus offenporigen Polyurethanschaum eines Raumgewichts, das zwischen 20 und 50 kg/m³ beträgt. Auf seiner Deckfläche 4 (Fig. 3) sind zur Bildung der Scheuerfläche 2 erhaben vorstehende, durchgehend ausgebildeten Leisten 3 angeordnet, die zwei einander senkrecht durchschneidende Leistenscharen bilden. Die Leistenscharen bilden einen einstückigen Bestandteil des den Reinigungskörper im Bereich der Oberseite bildenden Schaumstoffkörpers 1. Sie sind durch einen schneidenden Prozeß abfallfrei erzeugt und in Fig.1 zur Verdeutlichung ihrer Lage schraffiert wiedergegeben. In Wirklichkeit sind die Leisten 3 bei dem beschriebenen Ausführungsbeispiel nicht durch scharkantige Linien von der Umgebung abgegrenzt sondern gleichmäßig in diese übergehend ausgebildet. Eine scharfkantig begrenzte Ausbildung ist aber selbstverständlich ebenfalls möglich.

Der gegenseitige Mittelpunktsabstand E bzw. E' einander benachbarter Leisten 3 beträgt innerhalb beider Leistenscharen 30 mm bei einer maximalen Höhe von 4 mm und einer minimalen Höhe von 2 mm, jeweils bezogen auf diejenige Stelle des Profils, an der dieses seinen größten Abstand von einer gedachten Verlängerung der Deckfläche 4 erreicht. Die Dimensionierung kann in Abhängkeit von der jeweiligen Verwendung variiert sein. Die Mittelpunktsabstände E, E'können ebenfalls eine voneinader abweichende Größe haben. Die räumliche Lage der Deckfläche 4 ist in Fig. 3 angedeutet.

Die Höhe der Leisten 3 variiert in Längsrichtung sinusförmig zwischen Stellen der größten und der kleinsten Höhe C,D. Außerdem haben die Leisten 3 in Querrichtung das Profil einer Glockekurve. Dieses ist am Fußpunkt durch die in Fig. 1 gezeigten Linien begrenzt. Die Linien sind auf dem Produkt nicht erkennbar insofern, als die Leisten 3 unter Vermeidung eines sprunghaften Richtungswechsels und scharfer Kanten in die Deckfläche 4 des Reinigungskörpers übergehen. Die Begrenzungsflächen der Leisten gehen außerdem an den Kreuzungsstellen abgerundet ineinander über. Die Bereiche unterschiedlicher Höhe C,D der Leisten 3 folgen somit in jeder Richtung regelmäßig wieder aufeinander, wobei die einzelnen Leisten 3 dennoch napfartige Eintiefungen 5 nach Art eines Waffelmusters umschließen. Der Boden der Eintiefungen 5 bestimmt die Lage der Deckfläche, über der sich die Leisten 3 erheben.

Die Eintiefungen 5 sind geeignet, größere Schmutzmengen, die von einer zu reinigenden Oberfläche abgelöst werden, weitgehend drucklos in sich aufzunehmen. Ein Einmassieren in die Porenstruktur des Reinigungskörpes wird trotz Ausübung erheblicher Andrückkräfte verhindert, weil diese Kräfte überwiegend von den Leisten 3 und insbesondere den Teilen der Leisten 3aufgenommen werden, in denen diese die größte Höhe C erreichen. Nach dem Überschreiten der seitlichen Randbegrenzung des zu reinigenden Objektes fallen daher abgelöste Schmutzbestandteile leicht aus den Eintiefungen 5 heraus, wonach die ursprüngliche Speicherkapazität wiederum erreicht wrd. Ein Festsetzen derartiger Schmutzbetanteile in inneren der Porenstruktur des Reinigungskörpes wird auf jeden Fall weitestgehend unterbunden.

Die Scheuerfläche 2 ist in ihrer Gesamtheit mit einer Beschichtung aus vernetztem, elastomerem Polyurethan überzogen, die im Zuge eines Imprägnierprozesses aufgebracht und nachfolgend verfestigt und unlösbar mit der Scheuerfläche 2 verbunden ist. Ihre mechanische Widerstandsfähigkeit und insbesondere ihre Abriebfestigkeit ist dadurch wesentlich vergrößert. Die Beschichtung ist in Querrichtung von Poren durchdrungen. Hieraus resultiert eine gute Wasserdurchlässigkeit in Richtung der zu bearbeitenden Oberläche, was es ermöglicht, in die offene Porenstruktur des Reinigungskörpers eingespeicherte Wassermengen durch Druck auf den Reinigungskörper leicht in Richtung der Vorderseite der Scheuerfläche 2 zu verlagern bzw. durch Nachlassen des Druckes einschließlich der während eines Reinigungsprozesses gelösten Schmutzbestandteile von der Scheuerfläche 2 wegzusaugen.

In die Beschichtung ist ein abrasiv wirkendes Granulat eingebettet, das aus Gummipartikeln und/oder einem Scheuermittel bestehen kann. Auch festhaftende Schmutzbestandteile können dadurch problemlos von einer zu reinigenden Oberfläche entfernt werden.

Der in der Darstellung wiedergegebene Reinigungskörper ist nur im Bereich von einer Scheuerfläche 2 mit Leisten 3 der erfindungsgemäßen Art versehen. Die Rückseite läßt sich dadurch zum Abtrocknen von bereits gereinigten Flächen verwenden. Sie wird bei dem gezeigten Ausführungsbeipiel durch einen aufkaschierten Viskoseschwamm 6 gebildet, der sich besonders gut für diese Zwecke eingnet. Abweichend hiervon ist es selbstverständlich ebenfalls möglich, bedarfsweise auch dessen Oberfläche eine zweite oder sogar auf noch mehr Flächen des Reinigungskörpers mit entsprechend oer abweichend ausgebildeten Leisten 3 zu versehen. Die gerade nicht im Gebrauch befindliche Scheuerfläche 2 wird bei solchen Ausführungen durch die sich beim normalen Reinigungsprozess mit einer anderen Scheuerfläche ergebenden Walkbewegungen des Reinigungskörpers sowie durch das druckfreie Aufnehmen und Ausströmen von Wasser durch die Porenstruktur der unbenutzen Scheuerfläche wieder vollständig von anhaftenden Schmutzbestandteilen befreit und für einen erneute Verwendung tauglich gemacht, ohne daß es hierfür eines besonderen Aufwandes bedarf.

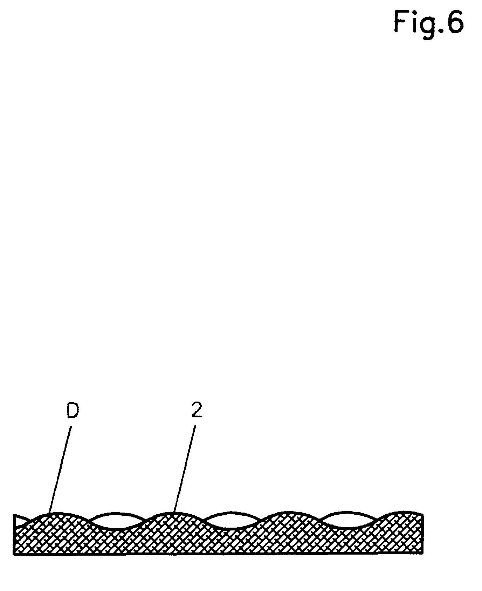

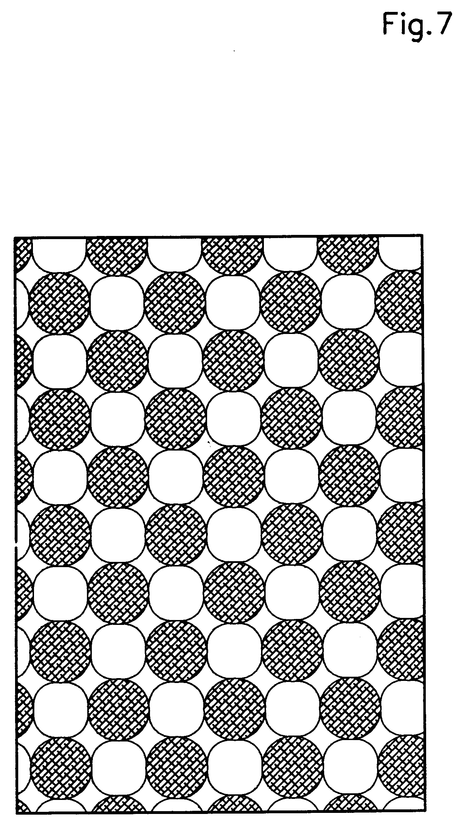

Die Fig. 6 und 7 zeigen einen Reinigungskörper in Gestalt eines Reinigungstuches aus Schaumstoff, das homogen aus einem einzigen Schaumstoffblock erzeugt und im Bereich der Unterseite durch eine ebene Fläche begrenzt ist. Die Oberseite wird aus einer Scheuerfläche 2 gebildet, die analog zu der vorstehend beschriebenen strukturiert ist, deren sich senkrecht durchschneidenden Leistenscharen indessen der Längsrichtung schräg, und zwar unter einem Winkel von 45° zugeordnet sind. Die in Längsrichtung hintereinander folgenden Stellen größter Höhe der Leisten 3 sind einander dadurch auf Lücke zugeordnet. Trotz verhältnismäßig großer, gegenseitiger Abstände der Stellen größer Höhe D der Leisten 3 wird dadurch quer zu der mit der Längsrichtung übereinstimmenden Arbeitsrichtung ein nahezu lückenloser Angriff auf die zu reinigende Oberfläche erreicht.

Wird der Reinigungskörper nach den Fig. 6 und 7 nicht genau parallel zu seiner Längsrichtung über die zu reinigende Fläche hinweg bewegt, sondern mehr oder weniger verdreht zu dieser Richtung, dann resultiert automatisch eine Verbesserung des gegenseitigen Überdeckungsgrades der jeweils durch die Stellen größter Höhe D gereinigten Streifen. Die derart schräge Zuordnung der Längsrichtung der Leisten 3 zur Bewegungsrichtung ist bei der normalen Durchführung eines Reinigungsvorganges fast immer erfüllt. Eine Anordnung und Ausbildung der Leisten 3 im Sinne der Figuren 6 und 7 wird aus diesem Grunde im Rahmen der vorliegenden Erfindung bevorzugt.

## Patentansprüche

1. Offenporiger, flexiber Reinigungskörper mit zumindest einer Scheuerfläche, die zumindest in einem Teilbereich mit über eine Deckfläche (4) mit einer Höhe (C,D) erhaben vorstehenden, kontinuierlich durchgehend ausgebildeten Leisten (3) versehen ist, dadurch gekennzeichnet, daß die Leisten (3) in Richtung ihres Verlaufs Bereiche einer voneinander abweichenden Höhe (C,D) haben.

2. Reinigungskörper nach Anspruch 1, dadurch gekennzeichnet, daß die Bereiche unterschiedlicher Höhe (C,D) regelmäßig wiederkehrend aufeinanderfolgen.

3. Reinigungskörper nach einem der Ansprüche 1 oder 2, dadurch gekennzeichnet, daß die Bereiche einer unterschiedlichen Höhe (C,D) gleichmäßig ineinander übergehend ausgebildet sind.

4. Reinigungskörper nach einem der Ansprüche 1 bis 3, dadurch gekennzeichnet, daß die Bereiche einer unterschiedlichen Höhe (C,D) sinusförmig aufeinanderfolgen.

5. Reinigungskörper nach einem der Ansprüche 1 bis 4, dadurch gekennzeichnet, daß Leisten (3) vorgesehen sind, die einander überkreuzen.

6. Reinigungskörper nach einem der Ansprüche 1 bis 5, dadurch gekennzeichnet, daß die Leisten (3), in Querrichtung betrachtet, durch geneigte Flächen begrenzt sind.

7. Reinigungskörper nach Anspruch 6, dadurch gekennzeichnet, daß die Flächen, in Querrichtung betrachtet, eine Wölbung haben.

8. Reinigungskörper nach Anspruch 7, dadurch gekennzeichnet, daß die Wölbung ein sinusförmig verlaufendes Profil hat.

9. Reinigungskörper nach einem der Ansprüche 1 bis 8, dadurch gekennzeichnet, daß das Verhältnis aus dem Mittelpunktsabstand (E, E') einander benachbarter Leisten (3) und deren maximaler Höhe (D) etwa 4 bis 12 beträgt.

10. Reinigungskörper nach einem der Ansprüche 1 bis 8, dadurch gekennzeichnet, daß das Verhältnis aus den gegenseitigen Mittelpunktsabständen (E,E') einander benachbarter Leisten (3) und deren maximaler Höhe (D) etwa 6 bis 9 beträgt.

11. Reinigungskörper nach einem der Ansprüche 1 bis 10, dadurch gekennzeichnet, daß die Scheuerfläche (2) mit einer die Abriebbeständigkeit verbessernden, flexiblen Beschichtung versehen ist.

12. Reinigungskörper nach Anspruch 11, dadurch gekennzeichnet, daß die Beschichtung aus elastomerem Polyurethan besteht.

13. Reinigungskörper nach Anspruch 12, dadurch gekennzeichnet, daß die Beschichtung im flüssigen Zustand auf die Scheuerfläche aufgebracht und nachfolgend verfentigt ist.

14. Reinigungskörper nach einem der Ansprüchen 10 bis 13, dadurch gekennzeichnet, daß in die Beschichtung Partikel einen Scheuermittels eingebettet sind.

15. Reinigungskörper nach einem der Ansprüche 10 bis 14, dadurch gekennzeichnet, daß die Beschichtung flüssigkeitsdurchdurchlässig ist.

16. Reinigungskörper nach einem der Ansprüche 1 bis 15, dadurch gekennzeichnet, daß die Längsrichtung der Leisten (3) zu der Längsrichtung den Reinigungskörpers schräg angeordnet sind.

## Claims

1. An open-cell, flexible cleaning element with at least one scouring surface which, at least in one sub-region, is provided with continuous ribs (3) which project in a raised manner at heights (C, D) over a top surface (4), characterized in that, in the direction in which they run, the ribs (3) have regions of differing heights (C, D).

2. A cleaning element according to claim 1, characterized in that the regions of different heights (C, D) follow one after the other in a regularly repeating manner.

3. A cleaning element according to either of claims 1 and 2, characterized in that the regions of different heights (C, D) are designed to merge uniformly one into the other.

4. A cleaning element according to any one of claims 1 to 3, characterized in that the regions of different heights (C, D) follow one after the other sinusoidally.

5. A cleaning element according to any one of claims 1 to 4, characterized in that ribs (3) which cross over one another are provided.

6. A cleaning element according to any one of claims 1 to 5, characterized in that, as seen in the transverse direction, the ribs (3) are bounded by inclined surfaces.

7. A cleaning element according to claim 6, characterized in that, as seen in the transverse direction, the surfaces are curved.

8. A cleaning element according to claim 7, characterized in that the curvature has a sinusoidally running profile.

9. A cleaning element according to any one of claims 1 to 8, characterized in that the ratio of the centre-to-centre distance (E, E') between mutually adjacent ribs (3) to the maximum height (D) of the latter is approximately 4 to 12.

10. A cleaning element according to any one of claims 1 to 8, characterized in that the ratio of the centre-to-centre distances (E, E') between mutually adjacent ribs (3) to the maximum height (D) of the latter is approximately 6 to 9.

11. A cleaning element according to any one of claims 1 to 10, characterized in that the scouring surface (2) is provided with a flexible coating which improves the abrasion resistance.

12. A cleaning element according to claim 11, characterized in that the coating consists of elastomeric polyurethane.

13. A cleaning element according to claim 12, characterized in that the coating is applied to the scouring surface in the liquid state and is then set.

14. A cleaning element according to any one of claims 10 to 13, characterized in that particles of a scouring agent are embedded in the coating.

15. A cleaning element according to any one of claims 10 to 14, characterized in that the coating is permeable to liquid.

16. A cleaning element according to any one of claims 1 to 15, characterized in that the longitudinal direction of the ribs (3) is arranged obliquely in relation to the longitudinal direction of the cleaning element.

## Revendications

1. Corps nettoyeur souple, à pores ouverts avec au moins une surface de frottement qui comporte dans un sous-domaine au moins des nervures (3) proéminentes, en saillie, constituées en continu et de façon ininterrompue au-dessus d'un plateau (4) [p.8, lignes 19 à 22] avec une hauteur (C, D) [p.9, lignes 6 à 9], caractérisé en ce que les nervures (3) dans le sens de leur tracé ont des zones de hauteur différée (C,D) les unes par rapport aux autres.

2. Corps nettoyeur selon la revendication 1, caractérisé en ce que les zones de hauteur différenciée (C, D) se succèdent régulièrement de façon récurrente.

3. Corps nettoyeur selon une des revendications 1 ou 2, caractérisé en ce que les zones d'une hauteur différenciée (C, D) sont formées uniformément en se fondant l'une dans l'autre.

4. Corps nettoyeur selon une des revendications 1 à 3, caractérisé en ce que les zones d'une hauteur différenciée (C, D) se succèdent sous forme sinusoïdale.

5. Corps nettoyeur selon une des revendications 1 à 4, caractérisé en ce que des nervures (3) qui se croisent sont prévues.

6. Corps nettoyeur selon une des revendications 1 à 5, caractérisé en ce que les nervures (3), considérées dans le sens transversal, sont limitées par des plans inclinés.

7. Corps nettoyeur selon la revendication 6, caractérisé en ce que les surfaces, considérées dans le sens transversal, présentent une courbe.

8. Corps nettoyeur selon la revendication 7, caractérisée en ce que la courbure a un profil sous forme sinusoïdale.

9. Corps nettoyeur selon une des revendications 1 à 8, caractérisé en ce que le rapport entre l'écart des centres (E, E') de nervures adjacentes (3) et leur hauteur maximale (D) est d'environ 4 à 12.

10. Corps nettoyeur selon une des revendications 1 à 8, caractérisé en ce que le rapport entre les écarts des centres (E,E') opposés de nervures adjacentes (3) et leur hauteur maximale (D) est d'environ 6 à 9.

11. Corps nettoyeur selon une des revendications 1 à 10, caractérisé en ce que la surface de récurage (2) est prévue avec un revêtement souple améliorant la résistance à l'abrasion.

12. Corps nettoyeur selon la revendication 11, caractérisé en ce que le revêtement est composé de polyuréthanne élastomère.

13. Corps nettoyeur selon la revendication 12, caractérisé en ce que le revêtement est appliqué à l'état liquide sur la surface de récurage et est ensuite durci.

14. Corps nettoyeur selon une des revendications 10 à 13, caractérisé en ce que des particules d'un produit à récurer sont insérées dans le revêtement.

15. Corps nettoyeur selon une des revendications 10 à 14, caractérisé en ce que le revêtement est perméable aux liquides.

16. Corps nettoyeur selon une des revendications 1 à 15, caractérisé en ce que le sens longitudinal des nervures (3) a une disposition oblique par rapport au sens longitudinal du corps nettoyeur.
